# EUROPEAN PATENT APPLICATION

(11) **EP 0 685 490 A1**
(43) Date of publication of application: **06.12.1995**
(21) Application number: 95108643.8
(22) Date of filing: 15.12.1989
(51) Int. Cl.: C07K 14/475, C07K 1/16, C12P 21/02

(54) **Process for the purification of homogeneous platelet-derived endothelial cell growth factor from a platelet lysate**

(30) Priority: 20.12.1988 US 288056
(62) Divisional of application: 89123229.0
(71) Applicant: LUDWIG INSTITUTE FOR CANCER RESEARCH, New York, New York 10105 (US)
(72) Inventor: Heldin, Carl-Henrik, S-75263 Uppsala (SE); Miyazono, Kohei, Bunkyo-ku, Tokyo 113 (JP); Wernstedt, Christer, S-75263 Uppsala (SE); Hellman, Ulf, S-75123 Uppsala (SE); Takaku, Fumimaro, Nerima-ku, Tokyo 176 (JP); Ishikawa, Fuyuki, Bunkyo-ku, Tokyo 113 (JP)
(74) Representative: Ryan, Anne Mary

(57) **Abstract**

A process for the purification of homogeneous plate-derived endothelial cell growth factor (PD-ECGF) from a platelet lysate comprises subjecting the lysate to cation exchange chromatography on CM-Sephadex, pooling and subjecting the resulting active fractions to anion exchange chromatography on QAE-Sephadex, pooling and subjecting the resulting active fractions to ammonium sulfate precipitation, pooling and subjecting the resulting active fractions to anion exchange chromatography on DEAE-Sepharose, pooling and subjecting the resulting active fractions to high-performance hydroxylapatite column chromatography, pooling and subjecting the resulting active fractions to high performance hydrophobic column chromatography on an alkyl-Superose resin, and recovering said homogeneous platelet-derived endothelial cell growth factor. The process, which can be carried out in five steps, allows for purification of PD-ECGF that results in about 1,250,000-fold purification at a yield of about 14%.

## Description

This invention relates to a process for the purification of homogeneous platelet-derived endothelial cell growth factor from a platelet lysate.

Platelet-derived endothelial cell growth factor (PD-ECGF) is a 45 kDa endothelial cell mitogen that has been purified to homogeneity from human platelets. It does not bind to heparin and does not stimulate the proliferation of fibroblasts, in contrast to other endothelial mitogens of the fibroblast growth factor (FGF) family. PD-ECGF appears to be the only endothelial cell growth factor in human platelets and recent data indicate that it has angiogenic activity in vitro, i.e., the ability to stimulate the formation of new blood vessels and chemotactic activity, in vitro. The present invention provides a homogeneous PD-ECGF in substantially greater yields than available in the past.

Polypeptide growth factors play an important role in stimulating proliferation of target cells which are involved in both normal cellular processes and disease states. Some examples of processes that involve cell proliferation are, for instance, epidermal tissue replacement and immune system responses. Hemostasis, which is prevention of blood loss, is a continual process requiring that vascular tissue be constantly replaced. Growth of vascular endothelial cells has a central role in a variety of physiological and pathological processes, such as angiogenesis, wound healing, atherosclerosis, and tumor growth. In the case of vascular trauma, a complex series of events comes into operation to maintain hemostasis. Initial response to trauma includes activation of platelet plugging, blood coagulation, and eventually regrowth or repair of the damaged tissue. This final step must be activated at the proper time, place and in the proper tissue. One role for polypeptide growth factors is to mediate this response in both a temporal and tissue specific manner.

Peptide and polypeptide growth factors have been isolated from many sources. These include epidermal tissue, neutral tissue, platelets, placental tissue, and others. These peptide and protein factors are distinguished by a variety of properties including target cell specificity, heparin affinity or interaction, secretory properties, and physiochemical properties such as molecular weight, charge, heat stability, pH sensitivity, and susceptibility to reducing agents.

Endothelial cell growth is stimulated by a class of polypeptide growth factors known as fibroblast growth factors (FGFs). These mitogenic factors are characterized by their heparin-binding properties. Heparin is a powerful anticoagulant agent normally found in minute amounts in the circulatory system. The FGFs fall into two distinct protein classes, acidic and basic, and have been identified in neural and other tissues (Baird et al., 1986; Lobb et al., 1986; Thomas et al., 1986).

Recently, endothelial cell proliferation was shown to be stimulated by a novel polypeptide growth factor that was distinct from other known polypeptide growth factors derived from fresh human platelets (Miyazona et al., 1985a,b). This factor was originally called vascular endothelial cell proliferation factor and was later renamed platelet-derived endothelial cell growth factor PD-ECGF.

Platelets are a rich source of growth factors for a variety of hemopoietic and other tissues. Platelet-derived growth factors that have been identified and characterized include platelet-derived growth factor (PDGF) which stimulates fibroblast and vascular smooth muscle cell growth but has no effect on endothelial cells; transforming growth factor-α (TGF-α ) which is closely related to epidermal growth factor (EGF) and can stimulate growth of epidermal cells but not endothelial cells; transforming growth factor-β (TGF-β ) which synergistically stimulates fibroblast growth in the presence of EGF and is furthermore a potent inhibitor of endothelial cell growth; a hepatocyte growth factor; and platelet-derived endothelial cell growth factor (PD-ECGF), the subject of this invention.

A growth-promoting activity was partially purified and characterized by Miyazono et al. (1987). The following characteristics were used to distinguish this activity from that of previously identified growth factors: Cultured porcine vascular endothelial cells were stimulated to incorporate ³H thymidine into DNA in a dose-dependent manner upon treatment with a soluble lysate of fresh human platelets. The platelet lysate also promoted cellular proliferation by about 100% above a control culture treated with 1% fetal bovine serum. Fractionation of the lysate revealed that the activity appeared in the Mr 20,000 range on a Sephadex G-75 gel filtration column, stimulated ³H thymidine incorporation into DNA of porcine vascular endothelial cells but not NRK fibroblasts, was probably distinct from PDGF which ran at Mr 30,000 on this column and stimulated fibroblasts but not endothelial cells, was more potent when prepared from fresh platelets rather than from outdated platelets, was heat-and acid-labile, resistant to the reducing agent dithiothreitol and was sensitive to trypsin, guanidinium-HC1 and urea, the latter two being denaturants. Based on these characteristics it was concluded that the growth-promoting activity was a polypeptide growth factor distinct from any that had previously been identified in human platelets.

Angiogenesis is the formation of new capillary blood vessels by sprouting from existing vessels. It is an important process in wound healing and tumor growth. Certain polypeptide factors have been identified which stimulate this process by virtue of their mitogenic or chemotactic activity for endothelial cells.

Mitogens are substances that stimulate proliferation of cells and are usually small molecules such as phorbal esters, polysaccharides, peptides, proteins or combinations thereof. Taxis is the directed migration of cells in response to an environmental cue. Chemotaxis is thus a response to chemicals in the environment. For eukaryotic cells, known chemotactic factors include histamine, amino acids, peptides, and proteins,

Polypeptide factors that elicit angiogenic responses include FGFs, TGF-α, TGF-β, tumor neurosis factor and angiogenin (Folkman et al., 1987a,b; Frater-Schroder et al., 1987; Leibovich et al., 1987). These factors exhibit multiple effects on a wide variety of cell types. Unlike these factors, human platelet-derived endothelial cell growth factor specifically stimulates endothelial cells.

Endothelial cell proliferation inside larger blood vessels is important for the regeneration of damaged endothelium and probably plays a role in prevention of atherosclerosis. Atherosclerosis is a condition in which lipid-rich lesions or plaques develop on blood vessels and can lead to numerous vascuous diseases. Alteration in the endothelial cell layer is thought to be an early event in atherosclerosis. Platelets adhere to damaged endothelium cells and release mitogens to stimulate both endothelial and smooth muscle cell regeneration. PD-ECGF is among the mitogens that are released.

In disease associated with a reduction in platelet number, for example thrombocytopenia, PD-ECGF and other platelet mitogens and chemotactic factors may provide therapeutic benefit. Certain drugs can also induce platelet destruction or suppression. Thus, supplementing drug therapy with platelet mitogens may prevent untoward side effects.

The present source of PD-ECGF is fresh human platelets making purification of large amounts of this protein expensive and potentially risky given that human blood may contain infectious agents. Since PD-ECGF is heat labile, prior sterilization or pasteurization of blood will destroy its activity. To obtain quantities of PD-ECGF for further study and to provide therapeutic amounts, a more readily available source is desired. Cloning the PD-ECGF gene would allow the construction of expression vectors that produce large amounts of PD-ECGF.

The techniques of recombinant DNA technology are well established. While numerous manuals are available that outline the strategy and methodology for cloning genes, much of the work remains unpredictable; there is still no guarantee of success. Each cloning experiment can present its own special problems and pitfalls. A typical strategy for cloning eukaryotic genes is to isolate mRNA, to transcribe it into cDNA which is then inserted into a replicable vector, to transform a suitable host with the construct and to identify the desired clone by any number of means including functional activity or complementation, and screening with antibodies or oligonucleotides A problem peculiar to some eukaryotic genes is finding a tissue in a state of development or differentiation that is expressing the desired gene product, that is, containing the mRNA from the desired gene. The source of PD-ECGF is platelets which are enucleated cells and thus do not contain DNA or mRNA. Hence, to clone the gene for PD-ECGF a tissue or cultured cell line that produces PD-ECGF was needed to be found.

The present invention provides an improved method for the purification of platelet-derived endothelial cell growth factor to homogeneity from fresh human platelets. PD-ECGF is a 45 kDal endothelial cell mitogen that does not bind heparin and does not stimulate the proliferation of fibroblasts, in contrast to another class of endothelial cell mitogens which belong to the FGF family. PD-ECGF has angiogenic and chemotactic activity. With greater yields of PD-ECGF available, a partial amino acid sequence can be obtained from tryptic fragments as well as from V8-protease- and CNBR-derived fragments.

Our European Patent Application No. 89123229.0 (EP-A 0 377 855) relates to a nucleic acid molecule comprising a recombinant DNA molecule or a cDNA molecule platelet-derived endothelial cell growth factor and to a mammalian platelet-derived endothelial cell growth factor for use in preparing a therapeutic composition useful in treating wounds, atherosclerosis, angiogenesis, or thrombocytopenia.

The present invention provides a process for the purification of homogeneous platelet-derived endothelial cell growth factor from a platelet lysate comprising subjecting the lysate to cation exchange chromatography, pooling and subjecting the resulting active fractions to anion exchange chromatography, pooling and subjecting the resulting active fractions to ammonium sulfate precipitation, pooling and subjecting the resulting active fractions to anion exchange chromatography, pooling and subjecting the resulting active fractions to high-performance hydroxylapatite column chromatography, pooling and subjecting the resulting active fractions to high-performance hydrophobic column chromatography, and recovering said homeogeneous platelet-derived endothelial cell growth factor.

Fig. 1 illustrates chromatographic purification of human PD-ECGF on DEAE-Sepharose.

Fig. 2 illustrates chromatographic purification of human PD-ECGF on hydroxylapatite.

Fig. 3 illustrates chromatographic purification of human PD-ECGF on a Mono Q column.

Fig. 4 illustrates chromatographic purification of human PD-ECGF on a TSK-G4000 SW column.

Fig. 5 illustrates chromatographic purification of human PD-ECGF on a Superose 12 column. A) Column profile. B) SDS polyacrylamide gel of purified fractions of human PD-ECGF. C) SDS polycrylamide gel of pure PD-ECGF in the presence or absence of a reducing agent.

Fig. 6 illustrates chromatographic purification of human PD-ECGF on a high-performance hydroxylapetite column. A) Column profile. B) Analysis of fractions by SDS gel electrophoresis and silver staining.

Fig. 7 illustrates chromatographic purification of human PD-ECGF on an alkyl Superose column. A) Column profile. B) Analysis of fractions by SDS gel electrophoresis and silver staining. C) Analysis of the purified material by SDS electrophoresis and silver staining under nonreducing conditions.

Fig. 8 illustrates separation of tryptic peptide fragments of human PD-ECGF by narrow-bore reversed phase HPLC.

Fig. 9 shows the amino acid sequence of human PD-ECGF obtained by sequencing proteolytic fragments of PD-ECGF.

Fig. 10 illustrates an immunoblot analysis of a human placental extract.

Vascular endothelial cells make a functional monolayer between blood and underlying tissue. In a normal vessel wall, endothelial cells are known to exist in a quiescent growth state. Proliferation of endothelial cells is a key component to a number of biological processes such as wound repair, thrombosis, atheroscelorsis and tumor growth. Thus, factors that regulate endothelial cell proliferation play a key role in these conditions.

Factors with the ability to stimulate the formation of blood vessels are implicated as causative agents in angiogenesis of normal and malignant tissue, and this is reviewed by Folksman and Klagsburn (1987a). The most well-characterized factors in this category are FGFs which are a family of heparin-binding endothelial cell mitogens originally isolated from neural tissue but are also found in macrophages and other tissues. Another endothelial cell mitogen is PD-ECGF.

PD-ECGF growth-promoting activity can be assayed by determining the incorporation of ³H-thymidine into the DNA of endothelial cells. Cells typically used in the assay are porcine aortic endothelial cells with bovine aortic endothelial cells, and human umbilical endothelial cells also being stimulated by PD-ECGF. Other established, cultured endothelial cell lines can be used in the assay with porcine aortic cells being preferred for routine assays.

Growth-promoting activity of PD-ECGF is also assayed by measuring cell proliferation. In this assay, fresh cells are plated in culture and the actual number of cells present is determined and compared to a control that has not received PD-ECGF. The cultured cell lines suitable for this assay are the same as described in the ³H-thymine incorporation assay.

When assayed on FGF cultures PD-ECGF does not stimulate incorporation of 3H thymidine into DNA nor does it induce cell proliferation.

PD-ECGF from platelets, especially human platelets, has been purified in small quantities (Miyazono et al., 1987). It has thus far been characterized as a 45 kDal polypeptide that is heat and acid labile, resistant to reducing agents such as dithiothreitol and others, and sensitive to proteolytic degradation, especially by trypsin and other serine proteases, and any of a number of well characterized proteases. The availability of larger amounts of pure PD-ECGF allows at least a partial amino acid sequence to be obtained in order to facilitate the cloning of its gene.

The purification of PD-ECGF is complicated by the fact that PD-ECGF occurs in low quantities in platelets and that its biological activity is both heat and acid labile. The process described by Miyazono et al. (1987) is a seven step process using both conventional chromatography and Fast Performance Liquid Chromatography (FPLC). The present invention contemplates an improved purification procedure that results in higher purity and substantially greater yields of PD-ECGF.

The present purification process begins with a side fraction of a platelet lysate in the purification of PDGF. This fraction is obtained by cation exchange chromatography on CM-Sephadex. The first step is anion exchange chromatography in batch on QAE-Sephadex. Next, the pooled fractions are concentrated by ammonium sulfate precipitation at about 42% of saturation. The third step is also anion exchange chromatography but on DEAE-Sepharose. The next three steps are all chromatographic involving adsorption chromatography on hydroxylapatite, anion exhange chromatography on Mono Q column attached to an FPLC system (Pharmacia), and gel filtration on a TSK-G4000 SW column. The final step is hydrophobic interaction chromatography on a Superose 12 column.

The present invention is directed to a more rapid process having fewer steps for purification of PD-ECGF that results in about 1,250,000-fold purification at a yield of about 14%, preferably in five steps.

The process is the same as above up to and including the DEAE-Sepharose anion exchange chromatography. Other variations of the purification are also possible even up to this stage. For instance, the platelet lysate can be freshly prepared and need not be a side fraction in the purification of another factor such as PDGF. The chrmoatographic resins employed as described above are preferable for the purification process but other equivalent resins that are commercially available can be substituted with comparable results. Ammonium sulfate precipitation is also done at about 42% saturation; however, one skilled in the art can easily adjust this amount as necessary to precipitate the maximum amount of PD-ECGF activity.

The improvement in the purification process is a reduction in the number of steps following DEAE-Sepharose anion exchange chromatography and speeding the process by using more rapid chromatographic separation techniques that are provided by HPLC, FLPLC and the like. The present process provides homogeneous ECGF in higher yields and evidencing higher biological activity than previously available.

Specifically, the fractions obtained from the DEAE-Sepharose step are pooled and subjected to high performance affinity chromatography on an hydroxylapatite column. This is followed by a final purification step using high peformance hydrophobic interaction chromatography. The resin preferred for this step is alkyl-Superose but other hydrophobic resin are suitable. Such resins are also commercially available. One of ordinary skill in the art can readily determine other suitable resins.

Stimulation of growth and chemotaxis of endothelial cells are in vitro properties that PD-ECGF has in common with other known angiogenesis factors (e.g. the FGFs). In contrast to these, however, PD-ECGF shows target cell specificity for endothelial cells in proliferation as well as chemotaxis assays. Thus, it can specifically and directly induce endothelial responses that are crucial for angiogenesis. PD-ECGF is the only endothelial mitogen present in a platelet lysate and may thus be an important factor involved in platelet mediated processes such as wound healing and the vascularization of thrombi. In addition, it may be of considerable importance as an intraluminal factor capable of regulating endothelial cell turnover if released from platelets. Endothelial cell proliferation inside larger vessels is important for the regeneration of damaged endothelium and thus probably important for the prevention of atherosclerosis.

### EXAMPLES

### Cell Cultures and Media

Endothelial cells were collected from a fresh porcine aorta using collagenase digestion as described by Booyse et al. (1975). Cloning of the endothelial cells was performed by single cell platings as described originally by Puck et al. (1956). The endothelial cells were maintained in 25 cm² culture flasks in Ham's F-10 medium containing 10% fetal bovine serum (FBS) and antibiotics, and subcultured using 0.25% trypsin solution (Gibco) when the cells reached confluency. There was no evidence of transformation or loss of the endothelial monolayer under these conditions for more than six months. Normal rat kidney (NRK) fibroblasts were obtained by the method described by Duc-Nguyen et al. (1966) and subcultured in Dulbecco's modified Eagle medium containing 10% FBS.

### Cell Number Determination

For growth experiments, endothelial cells were subcultured at a plating density of 20,000 cells/dish in a 35-mm tissue culture dish (Corning) using Ham's F-10 medium containing 10% FBS. The cells were incubated for 24 h to allow for attachment, and then the medium was changed to the test media. The cells were removed from the dishes by incubating for 15 min. in trypsin-EDTA and cell number was determined in duplicate. The cultures were refed with fresh test media on Day 4.

### Preparation of Platelet Lysate

Blood was collected from normal volunteers in citrate-phosphate-dextrose solution, and fresh platelet-rich plasma was obtained by centrifugation at 160 g for 20 min at 20°C. The upper four-fifths portion of platelet-rich plasma was centrifuged at 1700 g for 20 min. at 20°C, and the platelet pellet was suspended in 10 mM Tris HCl (pH 7.4)/150 mN NaCl/0.01 % polyethylene glycol (PEG). The platelets were washed twice with the same buffer in the centrifuge and sonicated for 1 min. By this method, erythocyte and leukocyte contamination in the last platelet suspension was less than 0.1%. Sonicated platelets were centrifuged at 48,000 g for 30 min. at 4°C and the supernatants were used as platelet lysate. About 2 ml of platelet lysate was obtained from 200 ml of whole blood. Further operations were performed at 4°C, unless otherwise specified.

### Assay for Growth Promoting Activity

Growth promoting activity was determined by using porcine aortic endothelial cells as indicator cells, as previously has been described (Miyazono et al., 1985 a, b). Endothelial cells, cultured in Ham's F10 medium supplemented with 10% fetal bovine serum (Flow Laboratories) and antibiotics, were subcultured weekly at a ratio of 1 to 20. For mitogenic assay, the cells were trypsinized and re-plated sparsely (approximately 1 x 10⁴ cells per well) with 500 ul of Ham's F-10 medium containing 0.5% fetal bovine serum and antibiotics in 24-well tissue culture plates (16 mm diameter, Costar). After 24 h of incubation, the test samples were added to the wells. 18 h later, [³H]-thymidine (0.2 uCi/well; 6.7 Ci/mM, New England Nuclear) was added. After an additional 4 h, the cells were fixed with ice cold 5% (w/v) trichloroacetic acid for 20 min. The resulting precipitates were washed extensively with water and solubilized with 200 ul of 1 M NaOH. After mixing at room temperature for 20 min, 200 ul of 1 M HCL was added to the wells. [³H]-radioactivity was then determined in a liquid scintillation counter using 20 ml of Instagel (Packard) per sample.

### Purification of Platelet-Derived Endothelial cell Growth Factor from Human Platelets

Assay - Growth factor activity was monitored throughout the purification procedures using porcine aortic endothelial cells as target cells as described above.

Growth promoting activity on human foreskin fibroblasts was assayed by the method by Betsholtz and Westermark (1984).

General - During the purification, plastic utensils were used to decrease the loss of activity by adsorption to glass surfaces. All operations were performed at 4°C, unless otherwise specified. Protein concentration was determined by the dye fixation assay of Bradford (1976), unless otherwise specified.
Preparation of Platelet Lysate - For the purification of endothelial cell growth factor, a side fraction from the purification of platelet-derived growth factor (PDGF) from human platelets (Heldin et al., 1987) was used. The first step in the purification of PDGF from platelet pellets is a chromatography on CM-Sephadex. The cationic PDGF is adsorbed to this column. Since endothelial cell growth factor in human platelets is an anionic protein, it was not adsorbed to the CM-Sephadex column in the initial step of the purification of PDGF (Heldin et al., 1987). As expected, the flow-through fraction was found to contain growth promoting activity for endothelial cells, and was used as starting material in the purification. About 15-liter of the non-adsorbed fraction was processed at a time; this was derived from about 900 l of fresh human blood. The non-adsorbed fraction was stored in 5 l containers at -20°C for up to 10 years until used.
QAE-Sephadex Chromatography - The non-adsorbed fraction from CM-Sephadex chromatography was thawed and processed to QAE-Sephadex chromatography. Dry QAE-Sephadex gel (0.7 g/l; A-50, Pharmacia) was added and mixed by shaking overnight. The gel was then allowed to sediment and, after the non-adsorbed fraction was discarded, it was poured into a column (60 x 5 cm, Pharmacia). The column was washed with 4 1 of 75 mM NaCl in 10 mM phosphate, pH 7.4, and eluted with 2.5 1 of 250 mM NaCl in 10 mM phosphate, pH 7.4.

The bulk of the activity bound to this column and eluted between 75 and 250 mM NaCl at pH7.4. This resulted in a 25-fold purification estimated by the protein recovered, and an assumed recovery of 80% in this step. Since the growth promoting activity in the starting material was variable, maybe due to the presence of inhibitory substances (see below), the yield in this step could not be determined exactly.
Ammonium Sulfate Precipitation - Ammonium sulfate (247 g/1, 42 % of saturation) was added to the eluate of QAE-Sephadex chromatography. After equilibration for 2 h at 4°C, the sample was centrifuged at 2,075 x g for 15 min. The precipitate was collected by centrifugation and resuspended in 50 mM NaCl in 10 mM bis(2-hydroxylethyl) amino-tris(hydroxymethyl)methane (Bis-Tris), pH 7.0.

At neutral pH, 42% saturation of ammonium sulfate precipitated about 90% of the activity, while only 9% of protein was coprecipitated. This procedure led to a 10-fold purification with about 700 mg of protein remaining in 200 ml of volume.
DEAE-Sepharose Chromatography - The material obtained by ammonium sulfate precipitation (approximately 700 mg of protein in the volume of 200 ml) was treated with 5mM dithiothreitol at room temperature for 2 h. The sample was then dialyzed extensively against 50 mM NaCl in 10 mM Bis-Tris, pH 7.0, and applied to a column of DEAE-Sepharose CL-6B (40 ml; Pharmacia). The column was washed with 100 ml of 50 mM NaCl in 10 mM Bis-Tris buffer, pH 7.0, and eluted with a linear gradient (800 ml) of NaCl from 50 to 200 mM in 10 mM Bis-Tris, pH 7.0, at a flow rate of 120 ml/hr. Fractions of 20 ml were collected and analyzed for protein and growth promoting activity.

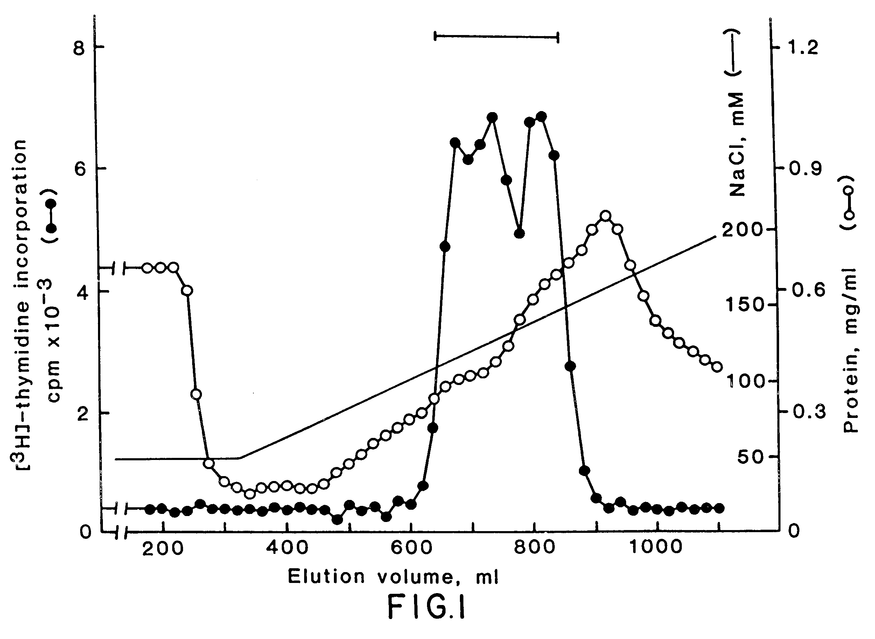

The endothelial cell growth promoting activity eluted at about 120-150 mM of NaCl at pH 7.0. This step led to a further 6-fold purification at a recovery of 50% (Fig. 1).

### Hydroxylapatite Chromatography

The active fractions from DEAE-Sepharose chromatography (approximately 60 mg of protein) were combined and loaded directly onto an 8 ml column of hydroxylapatite (Clarkson Chemical Co.: Williamsport, PA) equilibrated with 50 mM NaCl, 0.6 mM phosphate, pH 7.4. The column was washed with 80 ml of the same buffer, eluted with 50 mM NaCl, 15 mM phosphate, pH 7.4, followed by 200 mM phosphate, pH 7.4. Fractions of 8 ml were assayed for protein and growth promoting activity.

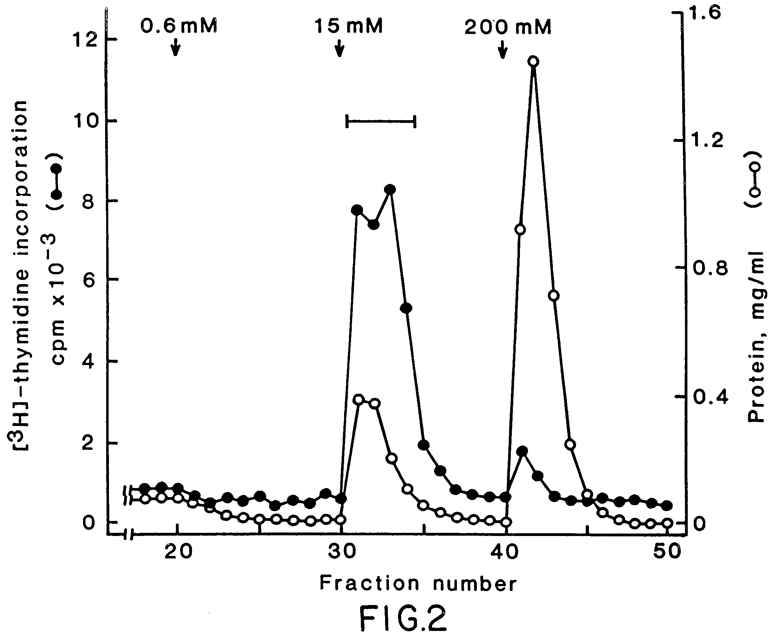

Hydroxylapatite chromatography gave a further 4-fold increase in specific activity (Fig. 2). Almost all the protein bound to the gel, and the growth promoting activity eluted at 15 mM phosphate, whereas the bulk of protein eluted at a higher concentration of phosphate. The recovery of the activity was about 55% in this step, and about 8 mg of protein remained.

Final purification of the growth factor was achieved using ion exchange and high-resolving gel chromatography columns attached to a fast-performance liquid chromatography (FPLC) apparatus.

### Mono Q Chromatography

The active fractions of the hydroxylapatite chromatography were pooled, centrifuged at 90,000 x g for 30 min at 4°C, and applied directly to a Mono Q column (HR 5/5, Pharmacia). This column and the following two (TSK-Gr000 SW and Superose 12), were attached to an FPLC-apparatus (Pharmacia) and operated at room temperature. The column was eluted at a flow rate of 1 ml/min with a gradient of 0-500 mM NaCl in 10 mM Bis-Tris, pH 7.0. Absorbance at 280 nm was monitored. Fractions of 1 ml were collected and tested for growth promoting activity.

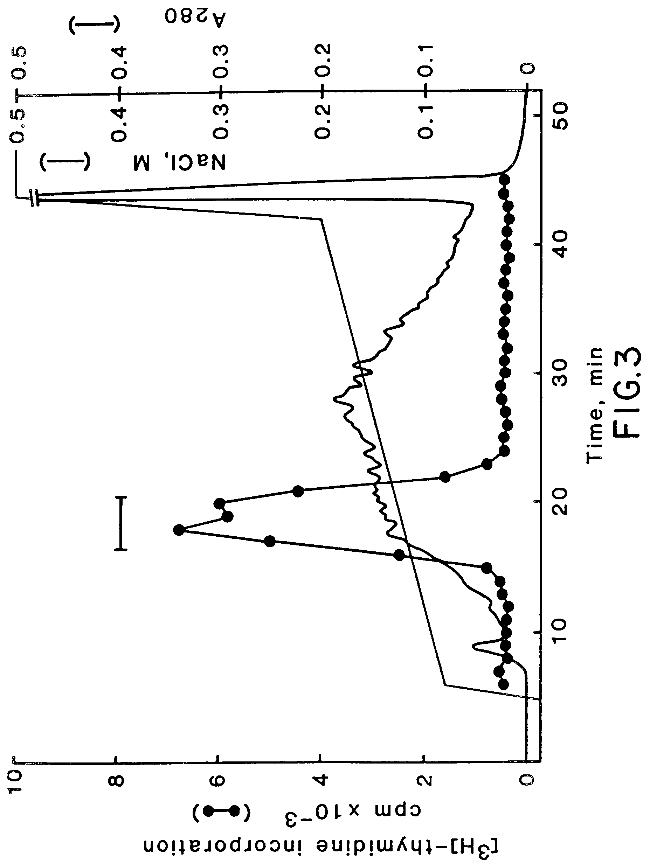

A single peak of growth promoting activity was obtained at 120 mM NaCl at pH 7.0 (Fig. 3). This step led to a further 4-fold purification with 50% of recovery.

### TSK-G4000 SW Gel Chromatography

The active fractions obtained from Mono Q column chromatography were pooled, lyophilized without prior dialysis and dissolved in 200 ul of water. The aliquot was then applied to a TSK-G4000 SW column (7.5 x 600 mm, LKB) with a precolumn (Ultropac column, 7.5 x 75 mm, LKB). The column was equilibrated with 100 mM phosphate, pH 6.5, and eluted at a flow rate of 500 ul/min. The absorbance of the column effluent was monitored at 280 nm. Each 500 ul fraction was collected and tested for growth promoting activity.

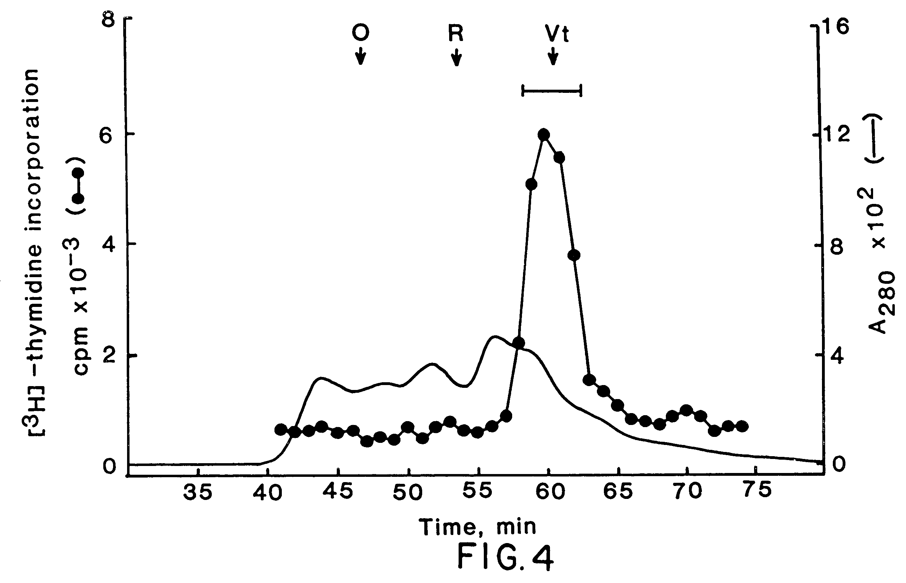

The result was a single broad peak of activity eluting anomalously late, at a position corresponding to a Mᵣ of less than 12,000 (Fig. 4). The recovery of the activity was 30% giving a 4-fold increase in specific activity.

### Superose 12 Gel Chromatography

The active fractions obtained from TSK-G4000 SW chromatography were pooled, dialyzed using Spectrapor dialysis tubing (Mᵣ cutoff, 3,500, Spectrum Medical Industries, Inc.) against 100 mM NaCl, 10 mM 4-(2-hydroxyethyl)-1-piperazine-ethanesulfonic acid (Hepes), pH 7.0, and then lyophilized. The material was resuspended in 100 ul of water and run on a Superose 12 column (HR 10/30, Pharmacia) in 200 mM NaCl, 10 mM Hepes, pH 7.0. The column was eluted at a flow rate of 400 ul/min and monitored with the absorbance at 280 nm. Fractions of 200 ul were collected and assayed for growth promoting activity.

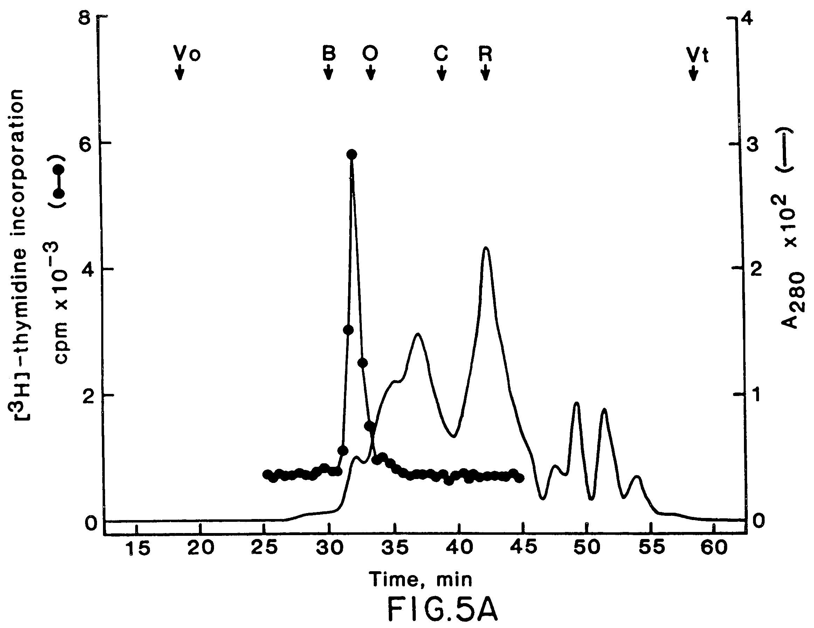

This procedure yielded a single sharp peak of bioactivity with an elution position between bovine serum, albumin and ovalbumin, well separated from the majority of protein (Fig. 5A). The increase in specific activity was 12-fold in this step at 30% recovery, giving 2 ug of pure material. The protein concentration in the purified fraction was estimated by comparing the intensity of the bands after sodium dodecyl sulfate (SDS)-gel electrophoresis and silver staining, with those of standards of bovine serum albumin.

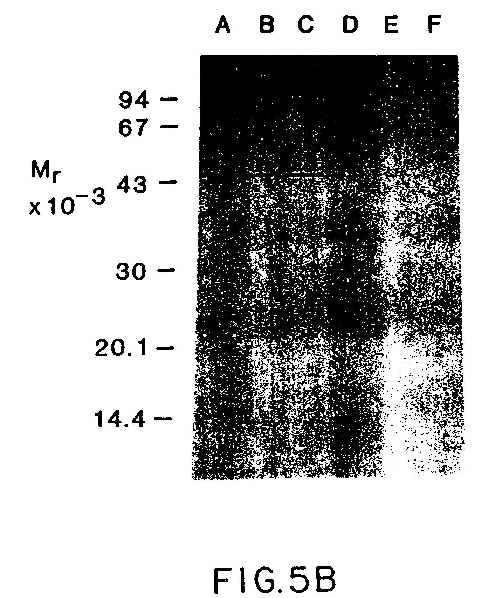

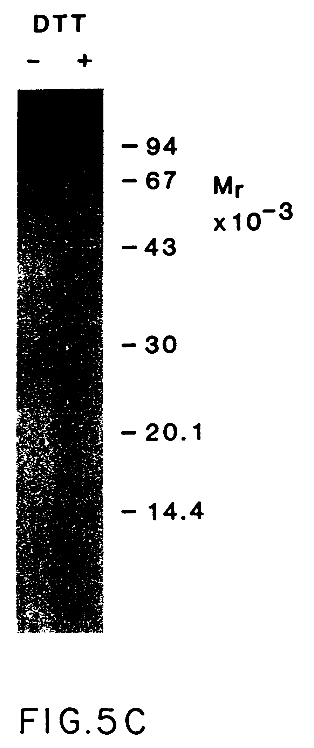

The protein compositions of the fractions from the Superose 12 chromatography step were analyzed by SDS-gel electrophoresis and silver staining (Fig. 5B). A homogenous protein with a Mᵣ of 45,000 exactly coeluted with the growth promoting activity, suggesting that it represents the growth factor. A similar mobility was observed when the samples were analyzed under non-reducing conditions (Fig. 5C), suggesting that the endothelial cell growth factor from platelets is composed of a single polypeptide chain.

A summary of the purification procedure from 200 g of platelet protein is shown in Table 1. The overall increase in activity was about 1,000,000-fold at a recovery of 1%.

**TABLE 1**

| Summary of the Purification of Platelet-Derived Endothelial Cell Growth Factor | | | | |
|---|---|---|---|---|
| purification step | protein (ug) | max stimulation (ng/mL) | purification (x-fold) | yield (%) |
| platelet lysate (flow-through from CM-Sephadex) | 200 000 000 | ^{a} | 1 | 100 |
| QAE-Sephadex | 8 000 000 | 1 000 000 | 20^{a} | 80^{a} |
| ammonium sulfate pptn | 700 000 | 100 000 | 200 | 70 |
| DEAE-Sepharose | 60 000 | 17 000 | 1 200 | 36 |
| hydroxyapatite | 8 000 | 4 000 | 5 000 | 20 |
| Mono Q | 1 000 | 1 000 | 20 000 | 10 |
| TSK-G4000 SW | 70 | 240 | 83 000 | 3 |
| Superose 12 | 2^{b} | 20 | 1 000 000 | 1 |

| | | | | |
|---|---|---|---|---|
| ^{a} The growth promoting activity in the platelet lysate was variable, maybe due to the presence of growth inhibitors (see, infra). Therefore, the specific activity of the starting material could not be accurately determined. The purification in the QAE steps was estimated at 20-fold, based on the amount of protein recovered and an assumed recovery of 80% of activity in this step. | | | | |
| ^{b} Protein concentration was estimated by the relative intensities of bands in silver stained SDS-gels. | | | | |

### Improved Purification Protocol for PD-ECGF

The initial steps of the purfication were performed as described above. As the next step, chromatography on an HPLC-grade hydroxylapatite column was used.

Chromatography on a High-Performance Hydroxylapatite Column. The material obtained from DEAE-Sepharose chromatography was filtered through a 0.22 um filter (Millipore) and loaded at room temperature onto a high-performance hydroxylapatite column (100 x 7.8 mm; BioRad) equipped with a guard column (50 x 4.0 mm; Bio-Rad). The column was preequilibrated with 1 mM phosphate buffer, pH 6.8, 50 mM NaCl, and 0.01 mM CaCl₂ and eluted at a flow-rate of 0.5 mL/min with a gradient of 1-100 mM phosphate, pH 6.8, 50 mM NaCl, and 0.01 mM CaCl₂. The column was then washed with 1 M phosphate buffer, pH 6.8, and 0.01 mM CaCl₂. Fractions of 1 mL were collected and tested for growth-promoting activity on porcine endothelial cells.

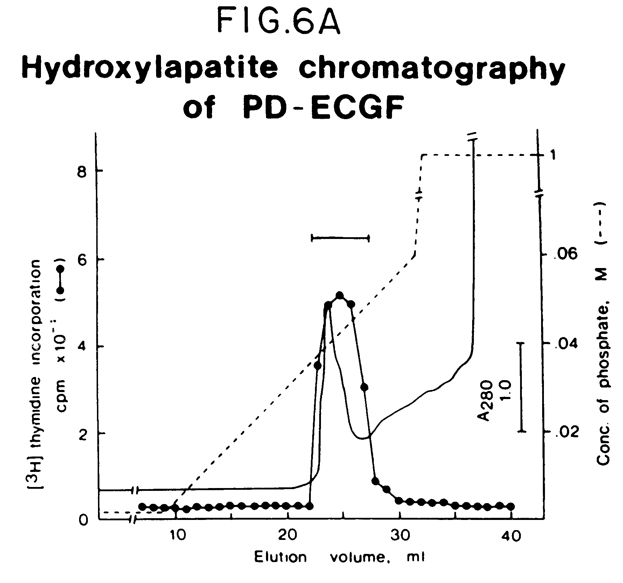

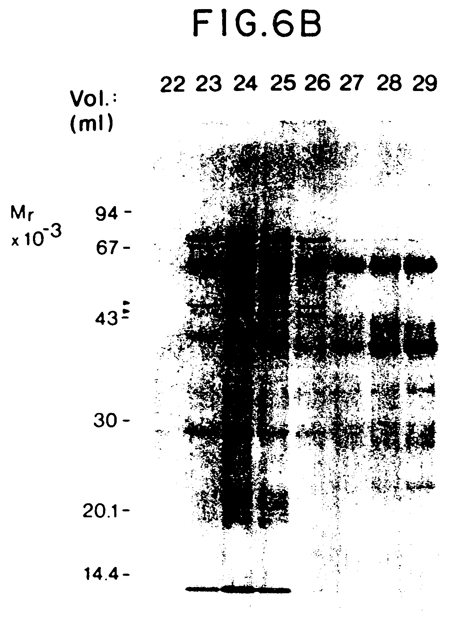

PD-ECGF bound to the gel in 1 mM phosphate buffer, pH 6.8, and was eluted with a linear gradient of phosphate (as indicated in Fig. 6A). The recovery of activity was about 32% in this step. Individual fractions from the chromatogram were analyzed by SDS gel electrophoresis and silver staining; the growth-promoting activity coeluted with two components of 46 and 44 kDa and some other components (Fig. 6B).

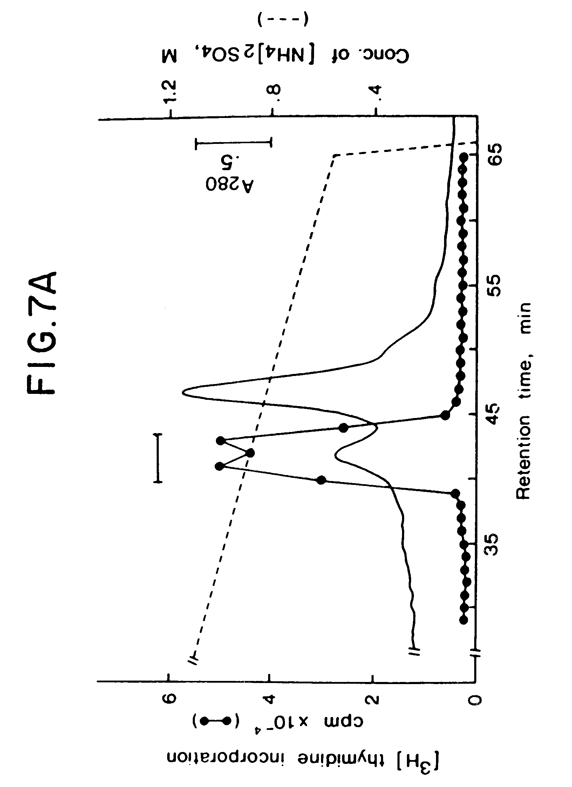

Chromatography on an Alkyl-Superose Column. The active fractions from the high-performance hydroxylapatite chromatography were pooled and mixed with an equal volume of 2.8 M ammonium sulfate (HPLC-grade, Bio-Rad) and 100 mM phosphate buffer, pH 6.8. The material was applied to an alkyl-Superose column (HR5/5, Pharmacia), preequilibrated with 1.4 M ammonium sulfate/100 mM phosphate buffer, pH 6.8, and eluted with a gradient of ammonium sulfate from 1.4 to 0 M in 100 mM phospate buffer, pH 6.8 as indicated in Figure 7A. The flow-rate was 0.5 ml/min and the column was operated at room temperature. The absorbance of the column effluent was monitored at 280 nm. Fractions of 500 ul were collected and assayed for grwoth-promoting activity.

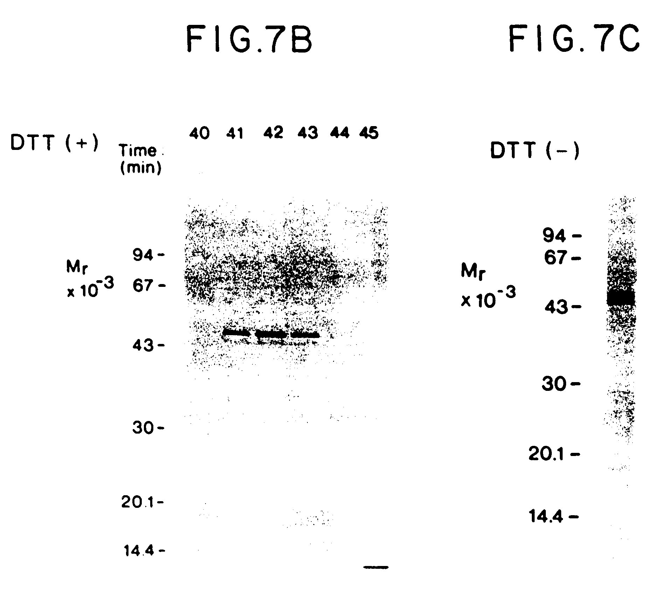

Final purification was achieved by hydrophobic chromatography using FPLC. The pooled fractions from the high-performance hydroxylapatite chromatography were applied to an alkyl-Superose column, equilibrated with 1.4 M ammonium sulfate/100 mM phosphate buffer, pH 6.8, and eluted with a decreasing gradient of ammonium sulfate in 100 mM phosphate buffer, pH 6.8. A single peak of growth-promoting activity was obtained at about 0.8 M ammonium sulfate (Fig. 7A). The increase in specific activity was 50-fold in this step at a recovery of 82%. The protein compositions of individual fractions of the alkyl-Superose chromatogram were analyzed by SDS gel electrophoresis and silver staining (Fig. 7B); again, the active fractions contained the principal two components of 46 and 44 kDa, indicating that PD-ECGF was essentially pure. When the purified material was analyzed under nonreducing conditions, bands of the same sizes were observed (Fig. 7C). Furthermore, a very faint band of Mᵣ 42,000 was reproducibly found. The proportion of these proteins in the silver-stained gels differed from preparation to preparation, but the 46- and 44-kDa components were always predominant.

A summary of the purification procedure starting from 300 g of platelet protein, corresponding to approximately 800-1000 L of human blood, is shown in Table 2. About 34 ug of pure PD-ECGF was obtained from each preparation. The material was purified 1,250,000-fold at an overall yield of about 14%.

**TABLE 2**

| Summary of the Purification of PD-ECGF^{a} | | | | |
|---|---|---|---|---|
| purification step | protein (ug) | max stimulation (ng/mL) | purification (x-fold) | yield (%) |
| platelet lysate (flow-through from CM-Sephadex) | 300 000 000 | ^{b} | 1 | ^{b} |
| QAE-Sephadex | 12 000 000 | 1 000 000 | 20^{b} | 80 |
| ammonium sulfate pptn | 800 000 | 80 000 | 250 | 58 |
| DEAE-Sepharose | 80 000 | 10 000 | 2 000 | 53 |
| high-performance hydroxyapatite | 2 000 | 800 | 25 000 | 17 |
| alkyl-Superose | 34^{c} | 16 | 1 250 000 | 14 |

| | | | | |
|---|---|---|---|---|
| ^{a} The results represent mean values of four individual preparations. | | | | |
| ^{b} The growth-promoting activity in platelet lysate was variable, maybe due to the presence of growth inhibitors (Miyazono, et al., 1987). Therefore, the purification in the QAE-Sephadex chromatography was estimated at 20-fold on the basis of the amount of protein recovered and an assumed recovery of 80% of activity (Miyazono, et al., 1987). | | | | |
| ^{c} Protein concentration was determined by amino acid analysis. | | | | |

### SDS-Gel Electrophoresis

SDS-gel electrophoresis was performed according to the method described by Blobel and Dobberstein, 1975. Briefly, samples were heated (95°C, 3 min.) with or without 10 mM dithiothreitol, and applied to a gradient gel consisting of 10-18% polyacylamide. The gel was fixed with glutaraldehyde after electrophoresis and then silver stained (18). Mᵣ markers (Pharmacia) containing phosphorylase b (94,000), bovine serum albumin (67,000), ovalbumin (43,000), carbonic anhydrase (30,000), soybean trypsin inhibitor (20,100) and a-lactalbumin (14,400) were used.

### Structural Analysis and Amino Acid Sequence of PD-ECGF

About 40 ug of pure PD-ECGF was reduced and pyridyl-ethylated, and then desalted using narrow-bore reversed phase HPLC. The material was then subjected to tryptic digestion followed by separation of the fragments on a narrow-bore reversed-phase HPLC column eluted in 0.1% trifluoroacetic acid with a gradient of acetonitrile.

For reduction and pyridyl-ethylation, PD-ECGF was first desalted on a C4 column and then dried in a Speedivac Concentrator and redissolved in 200 ul of 6 M guanidine-HCL, 0.25 M Tris-HCl, pH 8.5 and 2 mM EDTA, containing 100 ug of dithiothreitol. The solution was flushed with nitrogen for 20 s and left at room temperature for 3 h, at which time 2 ul of 4-vinyl-pyridine was added. After another three hours at room temperature, the sample was desalted by chromatography on the C4 column in the trifluoracetic acid/acetonitrile buffer system. The volatile solvent was removed as above and PD-ECGF was digested with TPCK-Trypsin (Sigma; enzyme to substrate ratio, 1/50 (w/w)) in 0.1 M ammonium bicarbonate, containing 2 M urea, for 4 h at 37°C. The tryptic fragments were immediately loaded onto a narrow bore reversed-phase HPLC. The chromatographic equipment consisted of a dual pump LKB system, adapted for narrow bore chromatography and equipped with a variable wavelength detector. The column temperature was kept at 35°C, the flow rate was 100 ul/min and the effluents were monitored at 220 nm. Fractions were collected manually in polypropylene tubes.

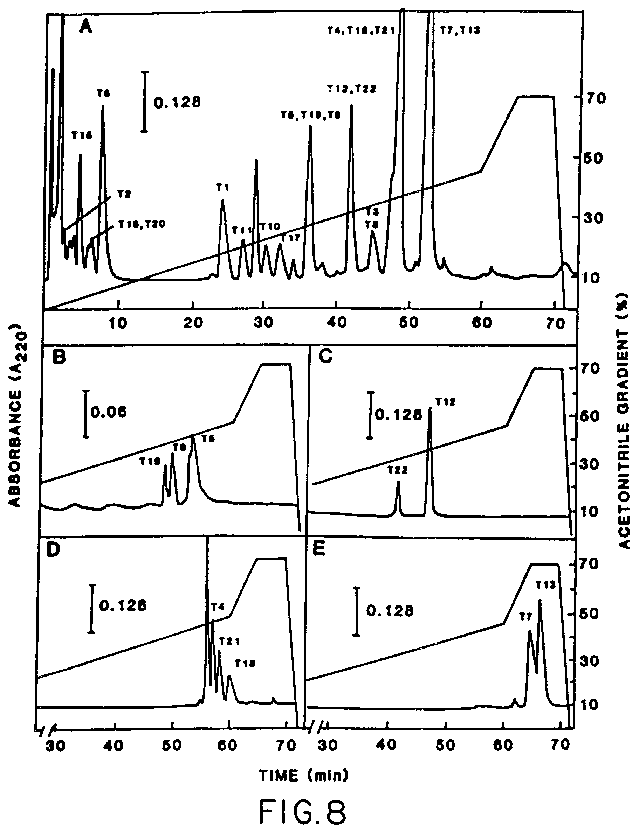

The separation of tryptic fragments of PD-ECGF is illustrated in Figure 8A. Approximately 40 ug of trypsin-digested PD-ECGF was loaded onto a C4 reversed phase column (Brownlee Aquapore BU-300; 2.1 x 30 mm and eluted with a linear gradient of acetonitrile in 0.1% trifuoracetic acid.

Non-homogenous peptides were rechromatographed under different conditions (examples are given in Fig. 8B-E); Figures 8B, 8D and and 8E illustrate rechromatography of the material under peaks T19, T9, T5; T4, T21, T18; and T7, T13, respectively, of Figure 8A. The samples were diluted three-fold with 0.1% trifluoracetic acid and loaded onto a Brownlee Spheri-5 RP-18 column (2.1 x 30 mm). The column was eluted with 0.1% trifluoracetic acid and a linear gradient of acetonitrile indicated. Figure 8C shows rechromatography of the peptides under peak T12, T22 of Figure 8A. The column used was the same as in Figure 8A, but it was eluted with 0.15 M NaCl in Milli-Q water with a gradient of acetonitrile as indicated.

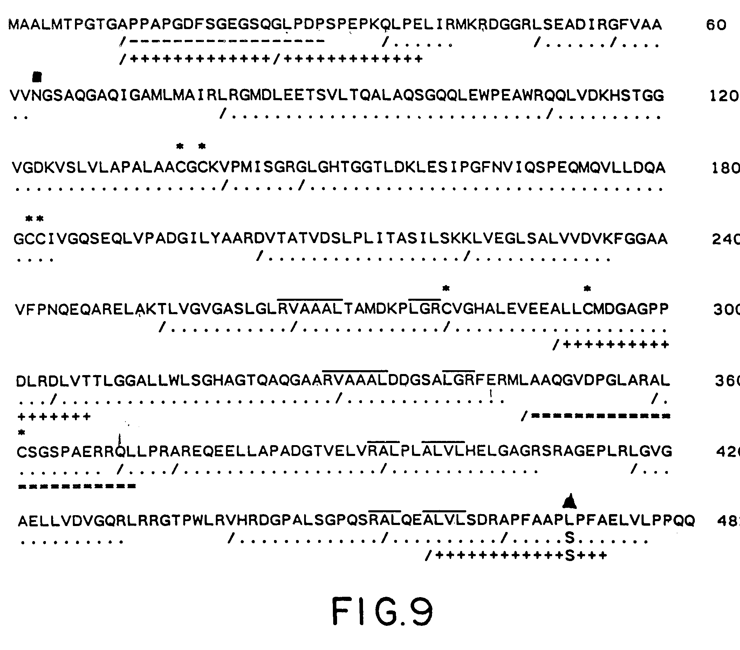

Single peptides were then subjected to N-terminal amino acid sequencing using a gas phase sequencer. (Applied Biosystems Protein Sequencer, model 470A, with an on line PTH-analyzer, model 120A). Sequence information from a total of tryptic peptides was obtained (Fig. 9). Additional sequence information was obtained by N-terminal sequencing of intact PD-ECGF, and by analysis of fragments obtained by digestion with CNBr of Staphylococcal V8 protease, using similar amounts of starting material.

In Fig. 9, tryptic fragments are indicated by (/...) and designations refer to peaks in Fig. 1. N-terminal sequence (/---), Staphylococcus protease V8 fragments (/+++) and a CNBr fragment (/===) of PD-ECGF are also indicated, as well as cysteine residues (*), a potential N-glycosylation site (#), and a possible site of polymorphism (@). Internal repeats are overlined.

### Preparation of Antibodies to PD-ECGF

To aid in cloning of cDNA for PD-ECGF, a specific polyclonal antiserum against PD-ECGF was raised and used in immunoblotting to localize a source of production of PD-ECGF.

The antiserum was prepared by diluting pure PD-ECGF to a concentration of 20 ug/ml in 10 mM phosphate buffer,, pH 7.4 with 150 mM NaCl. Then, ten micrograms of pure PD-ECGF was mixed with an equal volume of Freund's complete adjuvant and injected intramuscularly into a rabbit. The rabbit was boosted 2 weeks later with 10 ug of PD-ECGF in Freund's incomplete adjuvant. After that, the rabbit was boosted every 2-3 weeks with 5 ug of PD-ECGF in Freund's incomplete adjuvant. The immunoglobulin fraction was purified by applying 4 mL of immune serum to a 2-mL column of protein A-Sepharose (Pharmacia) equilibrated with 100 mM phosphate buffer, pH 7.4. The column was washed with the same buffer and then eluted with 50 mM citrate buffer, pH 3.0. The eluate from the column was rapidly neutralized with 1 M Tris-HCl, pH 7.4.

Since PD-ECGF occurs in platelets, several hematopoietic cell lines were tested, but none was found that synthesized PD-ECGF. A strong 45 kDa band was, however, found when an extract of a term human placenta was analyzed by immunoblotting. The human term placenta was minced and homogenized with 4 volumes of phosphate buffer saline (PBS). To the homogenate was added ammonium sulfate at 28% saturation; after centrifugation additional ammonium sulfate was added to the supernatant, to 42% saturation. The precipitate, recovered after centrifugation, was dissolved in PBS. Samples of 100 ng of PD-ECGF, purified from human platelets as described previously, and 375 ug of ammonium sulfate fractionated human placenta extract were subjected to SDS-gel electrophoresis in 10-18% gradient polyacrylamide gel under reducing conditions, and transferred to a nitrocellulose membrane in a buffer containing 20% ethanol, 150 mM glycine, and 20 mM Tris-HCl, pH 8.4, at 200 mA. The nitrocellulose membrane was incubated in 150 mM NaCl, 10 mM Tris-HCl, pH 7.4, 10% bovine serum albumin to block non-specific binding, incubated in a 1:50 dilution of a specific rabbit PD-ECGF antiserum, washed twice with 150 mM NaCl, 10 mM Tris-HCl, pH 7.4, and twice with 150 mM NaCl, 10 mM Tris-HCl, pH 7.4, 0.05% Triton X-100. The nitrocellulose membrane was then incubated with ¹²⁵I-labeled Staphylococcal protein A (5 x 10⁵ cpm/ml), and washed as described above. Blots were subjected to autoradiography. PD-ECGF purified from platelets occurs as a doublet of about 45 kDa, probably due to proteolysis during preparation.

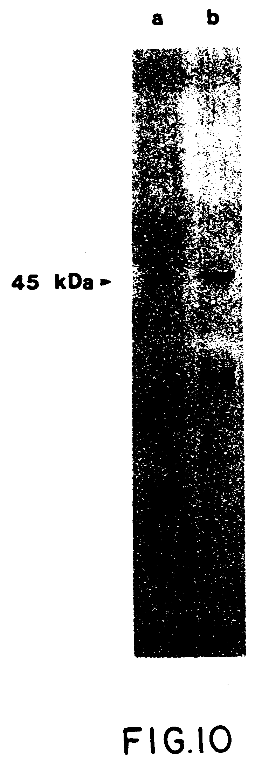

Figure 10 illustrates an immunoblot analysis of a human placenta extract (lane a) and PD-ECGF purified from human platelets (lane b) using the PD-ECGF polyclonal antiserum.

### BIBLIOGRAPHY

Baird, A., Esch, F., Mormede, P., Ueno, N., Ling, N., Bohlen, P., Ying, S.-Y., Wehrenberg, W.B. & Guillemin, R. (1986) in Recent Progress in Hormone Research 42: 143-205.
Betsholtz, C. & Westermark, B. (1984) J. Cell Physiol. 118: 203-210.
Blobel, G. & Dobberstein, B. (1975) J. Cell Biol. 67: 835-851.
Booyse, F.M., Sedlak, B.J. & Rafelson, M.E. (1975) Thromb. Diathes. Haemorrh. 34: 825.
Bradford, M.M. (1976) Anal. Biochem. 72: 248-254.
Duc-Nguyen, H., Rosenblum, E.N. & Zeigel, R.F. (1966) J. Bacteriol. 92: 1133.
Folkman, J. & Klagsbrun, M. (1987a) Science 235: 442-447.
Folkman, J. & Klagsbrun, M. (1987b) Nature 329: 671-672.
Frater-Schroder, M., Risau, W., Hallmann, R., Gautschi, P. & Bohlen, P. (1987) Proc. Nat. Acad. Sci. U.S.A. 84: 5277-5281.
Heldin, C.-H., Johnsson, A., Ek, B., Wennergren, S., Ronnstrand, L., Hammacher, A., Faulders, B., Wasteson, A. & westermark, B. (1987) Methods Enzymol. 147: 3-13.
Leibovich, S.J., Polverini, P.J., Shepard, H.M., Wiseman, D.M., Shively, V. & Nuseir, N. (1987) Nature 329: 630-632.
Lobb, R.R., Harper, J.W. & Fett, J.W. (1986) Anal. Biochem. 154: 1-14.
Miyazono, K., Okabe, T., Urabe, A., Yamanaka, M. & Takaku, F. (1985a) Biochem. Biophys. Res. Comm. 126: 83-88.
Miyazono, K., Okabe, T., Ishibashi, S., Urabe, A. & Takaku, F. (1985b) Exp. Cell Res. 159: 487-494.
Miyazono, K., Okabe, T., Urabe, A., Takaku, F. & Heldin, C.-H. (1987) J. Biol. Chem. 262: 4098-4103.
Puck, T.T., Marcus, P.I. & Cieciura, S.J. (1956) J. Exp. Med. 103: 273.
Thomas, K.A. & Gimenez-Gallego, G. (1986) Trends Biochem. Sci. 11: 81-84.

## Claims

1. A process for the purification of homogeneous platelet-derived endothelial cell growth factor from a platelet lysate comprising subjecting the lysate to cation exchange chromatography, pooling and subjecting the resulting active fractions to anion exchange chromatography, pooling and subjecting the resulting active fractions to ammonium sulfate precipitation, pooling and subjecting the resulting active fractions to anion exchange chromatography, pooling and subjecting the resulting active fractions to high-performance hydroxylapatite column chromatography, pooling and subjecting the resulting active fractions to high-performance hydrophobic column chromatography, and recovering said homogeneous platelet-derived endothelial cell growth factor.

2. A process according to claim 1, wherein the cation exchange chromatography is carried out on CM-Sephadex.

3. A process according to claim 1 or 2, wherein the first anion exchange chromatography step is carried out on QAE-Sephadex.

4. A process according to any preceding claim, wherein the second anion exchange chromatography step is carried out on DEAE-Sepharose.

5. A process according to any preceding claim, wherein the high performance hydrophobic column chromatography is carried out on an alkyl-Superose resin.
